# EUROPÄISCHE PATENTANMELDUNG

(11) **EP 0 634 166 A1**
(43) Veröffentlichungstag der Anmeldung: **18.01.1995**
(21) Anmeldenummer: 94110734.4
(22) Anmeldetag: 11.07.1994
(51) Int. Cl.: A61K 9/00, A61K 9/50, A61K 47/24, A61K 9/14

(54) **Arzneistoffzubereitungen aus umhüllten, schwerstwasserlöslichen Arzneistoffen für Inhalationsarzneiformen und Verfahren zu ihrer Herstellung**

(30) Priorität: 15.07.1993 DE 4323636
(71) Anmelder: HOECHST AKTIENGESELLSCHAFT, D-65929 Frankfurt am Main (DE)
(72) Erfinder: Reul, Bernhard, D-61462 Königstein (DE); Petri, Walter, Dr., D-65195 Wiesbaden (DE)

(57) **Zusammenfassung**

Es werden Arzneistoffzubereitungen aus mikronisierten Teilchen von schwerstwasserlöslichen Arzneistoffen, welche dadurch gekennzeichnet sind, daß sie mit einem in Wasser micell-kolloidal löslichen, natürlichen, physiologisch verträglichen Ampho-Surfactant umhüllt sind sowie Verfahren zur Herstellung dieser Arzneistoffzubereitungen beschrieben. In ein entsprechendes Endbehältnis gebracht und nach Zusatz eines chlorfreien, teilfluorierten, druckverflüssigbaren Treibgases sind diese Zubereitungen zur Inhalation geeignet.

## Beschreibung

Die Erfindung betrifft Arzneistoffzubereitungen, enthaltend einen oder mehrere schwerstwasserlösliche(n) mikronisierte(n) Arzneistoff(e), der/die zusammen mit einem speziellen in Wasser micell-kolloidal löslichen Ampho-Surfactant lyophilisiert oder sprühgetrocknet wird/werden.

Die so umhüllten Arzneistoffe sind, eindosiert in Endbehälter, gegebenenfalls unter Zusatz von druckverflüssigbarem Treibgas, als Arzneiform zur Inhalation geeignet.
Zur Inhalation geeignete Arzneistoffzubereitungen schwerstwasserlöslicher Arzneistoffe sind beschrieben.
So betrifft EP-A-0 465 841 schnellwirksame Inhalationsarzneiformen des schwerstwasserlöslichen Furosemids oder Piretanids.

Das dort beschriebene Pulverinhalat (= DPI) ist lokal schlecht verträglich; die schwere Benetzbarkeit der schwerstwasserlöslichen Arzneistoffe führt zu Reizungen auf der Lungenschleimhaut (vgl. Thoma, Karl, Aerosole, Frankfurt/Main, 1979, Seite 154-160).
Zusätzlich verstärken die hohen Hilfsstoffanteile (bis zu 50% Lactose), die zur Verminderung der bekannten Adhäsions- und Agglomerationseigenschaften schwerstwasserlöslicher, mikronisierter Arzneistoffe unbedingt erforderlich sind, die Reizerscheinungen auf der Lungenschleimhaut.

Das ebenfalls in EP-A-0 465 841 beschriebene Dosieraerosol (= MDI)ist -ein Suspensionsaerosol und besitzt folgende Nachteile:
1. das als Treibmittel eingesetzte druckverflüssigbare Treibgasgemisch ist auf reiner FCKW-Basis aufgebaut, d.h. aufgrund der neuen Gesetzgebung künftig nicht mehr verwendbar,
2. die eingesetzten, in der FCKW-Basis löslichen Suspendierhilfsstoffe mit oberflächenaktiven Eigenschaften und mit physiologischer Akzeptanz, wie Ölsäure, Benzalkoniumchlorid, Lecithin und Sorbitantrioleat, sind in den künftig nur noch erlaubten chlorfreien, teilfluorierten, druckverflüssigbaren Kohlenwasserstoffen (= HFKW) schwer- bzw. unlöslich, d.h. unter Beibehaltung der üblichen Herstellungsverfahren künftig nicht mehr einsetzbar.

In der Literatur sind, außer den unter 2. genannten, weitere oberflächenaktive Substanzen beschrieben, die sich als Suspendierhilfsstoffe für Suspensionsdosieraerosol-Formulierungen in dem HFKW-Medium lösen (vgl. z.B. WO 91/11173, WO 92/00062 und EP-A-0 504 112).
Diese weiteren, oberflächenaktive Substanzen sind aufgrund ihrer bisher unbekannten Inhalationstoxizität für Inhalationsarzneiformen noch nicht zugelassen.

Weiterhin ist in der Literatur ein Verfahren zur Beschichtung (Coating) von bestimmten Arzneistoffen mit den bisher üblichen Suspendierhilfsstoffen Ölsäure, Benzalkoniumchlorid, Lecithin und Sorbitantrioleat und die Verwendung der so gecoateten Arzneistoffe in Suspensionsdosieraerosol-Formulierungen auf FCKW- oder HFKW-Basis (vgl. WO 92/08447), gegebenenfalls mit Ethanol als Cosolvent (vgl. WO 92/08446) beschrieben.

Das dort beschriebene Verfahren ist jedoch für eine gleichmäßige, lückenlose und gewichtskonstante Beschichtung eines jeden mikronisierten Arzneistoffteilchens aus physikalischen Gründen ungeeignet; durch das Verdampfen des Lösungsmittels kommt es zwangsläufig zu einem Chromatographie-Effekt, der neben der Entstehung unterschiedlich dick beschichteter Arzneistoffteilchen auch noch zu Kornvergröberungen infolge von Verklebungen führt.

Darüberhinaus können nur Arzneistoffe beschichtet werden, die in den beschriebenen nicht polaren Lösungsmitteln, wie z.B. Iso-Pentan und Trichlorfluormethan schwerstlöslich bzw. limitiert löslich sind, d.h. vorzugsweise hydrophile Arzneistoffe.
Ebenso können nur Hilfsstoffe eingesetzt werden, die in den beschriebenen Lösungsmitteln löslich sind, d.h. vorzugsweise lipophile Hilfsstoffe.

Beschrieben ist auch der Einsatz von chlorfreien, teilfluorierten, druckverflüssigbaren Kohlenwasserstoffen (= HFKW) wie z.B. Heptafluorpropan (1,1,1,2,3,3,3-Heptafluorpropan, R 227) oder Tetrafluorethan (1,1,1,2-Tetrafluorethan, R 134a) als Treibmittel für technische und kosmetische Aerosole (vgl. EP-A-0 384 371) und auch für pharmazeutische Aerosole (vgl. EP-B-0 372 777), gegebenenfalls in Mischung mit halogenfreien Kohlenwasserstoffen (vgl. WO 91/11496) oder in Mischung mit Ethanol (vgl. DE-A-41 23 663).

Der Erfindung lag die Aufgabe zugrunde:
a. die bekannt schlechte Verträglichkeit von schwerstwasserlöslichen, auf der Lungenschleimhaut schwer benetzbaren und dadurch Reizungen auslösenden Arzneistoffe zu verbessern,
b. Inhalationsarzneiformen mit schwerstwasserlöslichen Arzneistoffen in Form von Pulverinhalaten bzw. Suspensionsdosieraerosolen mit dem chlorfreien, teilfluorierten, druckverfüssigbaren Treibgas (R 227) zu formulieren,
c. die Substanzbelastung in der Lunge durch Formulierungsbestandteile so klein wie möglich zu halten.

Diese Aufgabe wird dadurch gelöst, daß eine feste, stabile Kombination bestehend aus schwerstwasserlöslichem, mikronisiertem Arzneistoff und einem in Wasser micell-kolloidal löslichen, natürlichen Ampho-Surfactant gefunden wurde. Diese Kombination wird im folgenden auch als Arzneistoff-Hilfsstoff-Paar (= AHP) bezeichnet.
Ein Ampho-Surfactant ist ein in Wasser micell-kolloidal löslicher, natürlicher, physiologisch verträglicher Ampholyt, analog zum körpereigenen "Surfactant " (vgl. Mutschler, Ernst Arzneimittelwirkungen, 6. Auflage, Stuttgart, 1991 und Keidel, Wolf-Dieter Physiologie, 6. Auflage, Stuttgart - New York, 1988).

Micell-kolloidal löslich bedeutet, daß der Ampholyt sowohl Micellen als auch Kolloide ausbildet (0,1 bis 500 nm).

Mikronisierter Arzneistoff bedeutet, daß seine Teilchengröße zur Inhalation geeignet ist, d.h. kleiner 5 µm ist.

Die Erfindung betrifft daher eine Arzneistoffzubereitung enthaltend einen schwerstwasserlöslichen, mikronisierten Arzneistoff oder ein schwerstwasserlösliches, mikronisiertes Arzneistoffgemisch, welche dadurch gekennzeichnet ist, daß der Arzneistoff oder das Arzneistoffgemisch mit einem in Wasser micell-kolloidal löslichen, natürlichen, physiologisch verträglichen Ampho-Surfactant umhüllt ist.

Die Erfindung betrifft weiter ein Verfahren zur Herstellung des AHP, d.h. Umhüllung des Arzneistoffes oder des Arzneistoffgemisches, das dadurch gekennzeichnet ist, daß man den schwerstwasserlöslichen, mikronisierten Arzneistoff bzw. das Arzneistoffgemisch in der wässrigen, micell-kolloidalen Lösung des Ampho-Surfactant suspendiert und danach einer Sprühtrocknung oder Lyophilisation unterwirft.

Bei der Durchführung des erfindungsgemäßen Verfahrens wird der schwerstwasserlösliche Arzneistoff bzw. das Arzneistoffgemisch nach üblichen Methoden, z.B. Fällung und Mahlung, mikronisiert.

Trocknung durch Lyophilisation ist bevorzugt.

Das erfindungsgemäße feste, stabile AHP eignet sich zur Inhalation, wobei sowohl die Arzneiform Pulverinhalat (= DPI) als auch die Arzneiform Dosieraerosol (= MDI) in Betracht kommen.
Die Erfindung betrifft daher auch diese Arzneiformen, wobei das AHP zweckmäßigerweise in für Pulverinhalat geeignete Behälter, z.B. in eine Kapsel wie eine Gelatinekapsel, oder in eine Kartusche für Mehrfachdosiersysteme oder in für Dosieraerosole geeignete Behälter unter Zusatz eines druckverflüssigbaren Treibgases, abgefüllt wird.

Als Treibgase für die Arzneiform Dosieraerosol werden die Ozonschicht nicht schädigende, d.h. chlorfreie, teilfluorierte, druckverflüssigbare Kohlenwasserstoffe (HFKW) wie 1,1,1,2,3,3,3-Hepta-fluorpropan (= R 227) und 1,1,1 ,2-Tetrafluorethan (= R 134a) oder deren Mischung verwendet.

Als Ampho-Surfactant werden in Wasser micell-kolloidal lösliche, spezielle Phospholipidgemische mit einem Gehalt von Phosphatidylcholin von 20 - 95%, vorzugsweise von 40 - 80 %, eingesetzt.

Je nach eingesetztem Arzneistofftyp und Dosierung und je nach ausgewählter Arzneiform sind für das jeweilig optimale AHP unterschiedliche Ampho-Surfactanttypen (d.h. mit unterschiedlichen Phosphatidylcholingehalt) und Konzentrationen erforderlich.
Durch eine individuelle Anpassung variiert der Gehalt zwischen 0,01 - 10 % Ampho-Sufactant im AHP.

Als Arzneistoffe kommen alle zur Inhalation geeignete Substanzen in Betracht, die schwerstwasserlöslich sind (Löslichkeit in Wasser kleiner 0,01 %):
z.B.
- als Diuretika:: Furosemid, Azosemid, Piretanid, Bumetanid und Torasemid
- als Antimykotika:: Clotrimazol, Miconazol, Ketoconazol, Itraconazol, Bifonazol und Rilopirox
- als Antidiabetika:: Glibenclamid, Glimepirid und Insulin
- als Antiallergika:: ASS/Furosemid-Kombination
Durch das Verfahren der Lyophilisation erhält die hydrophobe Oberfläche der schwerstwasserlöslichen, mikronisierten Arzneistoffteilchen eine hydrophile gleichmäßige Umhüllung. Somit werden die bekannten Adhäsions- und Agglomerationseigenschaften mikronisierter Arzneistoffteilchen aufgehoben.

Durch die Verwendung eines speziellen natürlichen Phospholipidgemisches (Ampho-Surfactant), das chemisch mit dem vom Körper gebildeten, auf der Lungenschleimhaut vorliegendem Surfactant nahezu identisch ist, wird neben der guten lokalen Verträglichkeit auch eine hervorragende Benetzbarkeit erreicht. Somit werden die bekannten Reizwirkungen vermieden.

Das so hergestellte und zusammengesetzte AHP ist im besonderen Maße für den Einsatz in den Inhalationsarzneiformen Pulverinhalat und Dosieraerosol mit den Treibgasen R 227 und R 134a geeignet.
Für die Zubereitungen sind keine weiteren fießfähigkeitsverbessernden bzw. suspensionsstabilisierende Hilfsstoffe erforderlich. Daher ist die Substanzbelastung der Lunge äußerst gering.
Diese AHP-Zubereitungen sind pharmazeutisch einwandfrei, sie sind dosiergenau applizierbar und lagerstabil.

Zur weiteren Erläuterung der Erfindung werden folgende Beispiele genannt:

### Beispiel 1: Herstellung Arzneistoff-Hilfsstoff-Paar (AHP)

0,4 g Phospholipidgemisch (80% Phosphatidylcholin) werden mit 80,0 ml Wasser pro Injectione 24 Stunden bei Labortemperatur (ca. 23° C) unter Rollen dispergiert. In die entstandene schwach opaleszierende, micell-kolloidale Phospholipidlösung werden 20,0 g mikronisiertes Furosemid (Teilchengröße 98 % < 5µ) eingetragen und suspendiert. Die homogene, luftfreie Suspension wird im Schockverfahren eingefroren (ca. - 80°C) und anschließend 48 Stunden im Hochvakuum (13 Pa) bei Labortemperatur getrocknet.
Ausbeute: 19,2 g.
Das AHP fällt in Form eines lockeren, freifließenden Pulvers an, mit einem Phosphatidylcholingehalt von 1,6 % und einem Wassergehalt von < 0,1 %. Der Wirkstoffgehalt liegt bei 97 - 98 %.

### Beispiel 2: Herstellung AHP-Pulverinhalat

Das AHP (Zusammensetzung gemäß Beispiel 1) wird über ein Sieb (Maschenweite 0,315 mm) gegeben und in Hartgelatinekapseln Größe 2 zu je 20,4 mg entsprechend 20,0 mg Furosemid abgefüllt.
Mit Hilfe eines geeigneten handelsüblichen Applikator (z.B. "Spinhaler") ist das AHP einwandfrei applizierbar. Die anwendungstechnischen Eigenschaften entsprechen den üblichen pharmazeutischen Anforderungen.

### Beispiel 3: Herstellung AHP-Dosieraerosol

### Verfahren A

Das AHP (Zusammensetzung gemäß Beispiel 1) wird über ein Sieb (Maschenweite 0,315 mm) gegeben und in für Dosieraerosole geeignete Behälter (Alumonoblock-Dose, Vol. 22 ml) zu je 0,510 g AHP abgefüllt, die Behälter werden mit einem für Suspensionsdosieraerosole geeigenten Dosierventil verschlossen, anschließend werden je Behälter 13,49 g R 227 über das Dosierventil eingefüllt. Nach kurzem Schütteln oder nach Ultraschallbehandlung entsteht eine homogene stabile Suspension von AHP in R 227.

### Beispiel 4: Herstellung AHP-Dosieraerosol

### Verfahren B

0,715 kg AHP (Zusammensetzung gemäß Beispiel 1) werden in einem Druckkessel (Vol. 20 l) mit Rührwerk und Homogenisator vorgelegt und anschließend werden unter Druck 7,985 kg R 227 zugesetzt. Das Ganze wird homogenisiert. Es entsteht eine Suspension. Für Dosieraerosole geeignete Behälter (Alumonoblock-Dosen, Vol. 22 ml) werden leer mit einem für Suspensionsdosieraerosole geeigneten Dosierventil (Vol. 0,1 ml) verschlossen. Im ersten Füllvorgang werden nach bekannter Druckfülltechnik in die Behälter je 12,5 g der homogenen Suspension eindosiert. Im anschließenden zweiten Füllvorgang werden zum Spülen der Dosierventile die Behälterfüllungen mit je 1,5 g R 227 verdünnt.

Eine Dosis des nach Verfahren A bzw. B hergestellten Suspensionsdosieraerosols enthält pro Sprühstoß (0,1 ml) 5,1 mg AHP (Zusammensetzung gemäß Beispiel 1) entsprechend 5,0 mg Furosemid, 0,1 mg Phosphatidylcholin und 134,9 mg R 227.

### Beispiel 5: AHP-Herstellung

0,3 g Phospholipidgemisch (45% Phosphatidylcholin) werden mit 270,0 ml Wasser pro Injectione 24 Stunden bei Labortemperatur (ca. 23°C) unter Rollen dispergiert. In die entstandene micell-kolloidale Phospholipidlösung werden 30,0 g mikronisiertes Rilopirox (Teilchengröße 98% < 5µ) eingetragen und suspendiert.

Die homogene, luftfreie Suspension wird im Schockverfahren eingefroren (ca. -80°C) und anschließend 48 Stunden im Hochvakuum (13 Pa) bei Labortemperatur getrocknet. Ausbeute: 29,1 g.
Das AHP fällt in Form eines lockeren, freifließenden Pulvers an, mit einem Phosphatidylcholingehalt von 0,5 % und einem Wassergehalt von 0,1 %. Der Wirkstoffgehalt liegt bei 98 - 99 %.

### Beispiel 6: AHP-Dosieraerosol

Das AHP (Zusammensetzung gemäß Beispiel 5) wird über ein Sieb (Maschenweite 0,315 mm) gegeben und in für Dosieraerosole geeignete Behälter (Alumonoblock-Dose, Vol. 22 ml) zu je 0,404 g AHP abgefüllt, die Behälter werden mit einem für Suspensionsdosieraerosole geeigneten Dosierventil verschlossen, anschließend werden je Behälter 27,60 g R 227 über das Dosierventil eingefüllt. Nach kurzem Schütteln oder nach Ultraschallbehandlung entsteht eine homogene stabile Suspension von AHP in R 227.

## Patentansprüche

1. Arzneistoffzubereitungen mit mikronisierten Teilchen von schwerstwasserlöslichen Arzneistoffen, dadurch gekennzeichnet, daß sie mit einem in Wasser micell-kolloidal löslichen, natürlichen, physiologisch verträglichen Ampho-Surfactant umhüllt sind.

2. Arzneistoffzubereitungen gemäß Anspruch 1, dadurch gekennzeichnet, daß die Arzneistoffe mit einem Phospholipidgemisch mit einem Phosphatidylcholingehalt von 20 - 95 %, vorzugsweise von 40 - 80 %, umhüllt sind.

3. Verfahren zur Herstellung einer Arzneistoffzubereitung nach Anspruch 1 oder 2, dadurch gekennzeichnet, daß man den mikronisierten Arzneistoff in einer wässrigen, micell-kolloidalen Ampho-Surfactantlösung suspendiert und anschließend einer Sprühtrocknung oder Lyophilisation unterwirft.

4. Vorgefertigtes Aerosol, dadurch gekennzeichnet, daß es eine Zubereitung gemäß Anspruch 1 und als chlorfreies, teilfluoriertes, druckverflüssigbares Treibgas Heptafluorpropan (R227) oder Tetrafluorethan (R 134a) oder Mischung dieser beiden Stoffe enthält.

5. Verfahren zur Herstellung einer Inhalationsarzneiform gemäß Anspruch 4, dadurch gekennzeichnet, daß man die Arzneistoffzubereitung gemäß Anspruch 1
a. in entsprechende Endbehältnisse eindosiert, diese mit einem Dosierventil verschließt und die zur Bildung des Suspensionsaerosol erforderliche Menge des chlorfreien Treibgases zudosiert oder
b. aus der Arznestoffzubereitung gemäß Anspruch 1 in einem separaten, druckdichten Ansatzbehälter ein Suspensionsaerosol herstellt und nach Homogenisierung die fertige Suspension in das Endbehältnis abfüllt.

6. Verfahren zur Herstellung eines vorgefertigten Aerosols,dadurch gekennzeichnet, daß man die Arzneistoffzubereitung gemäß Anspruch 1 in geeignete Kapsel- oder Reservoirsysteme abfüllt.

7. Arzneistoffzubereitungen gemäß Anspruch 1 enthaltend Diuretika.

8. Arzneistoffzubereitungen gemäß Anspruch 1 enthaltend Furosemid.

9. Arzneistoffzubereitungen gemäß Anspruch 1 enthaltend Antidiabetika.

10. Arzneistoffzubereitungen gemäß Anspruch 1 enthaltend Antimykotika.

11. Arzneistoffzubereitungen gemäß Anspruch 1 enthaltend Rilopirox.
